# EUROPEAN PATENT SPECIFICATION

(11) **EP 3 463 528 B1**
(45) Date of publication and mention of the grant of the patent: **29.12.2021**
(21) Application number: 17802334.7
(22) Date of filing: 19.05.2017
(51) Int. Cl.: A61M 11/00, A61M 13/00, A61M 15/00, A61M 15/06, A61M 16/00, A61M 16/10

(54) **INHALER SPACER**
INHALATORABSTANDSHALTER
ÉLÉMENT D'ESPACEMENT D'INHALATEUR

(30) Priority: 23.05.2016 IN 201621017672
(43) Date of publication of application: 10.04.2019
(73) Proprietor: Cipla Limited, Mumbai 400 013 (IN)
(72) Inventor: MALHOTRA, Vidur, Mumbai 400014 (IN); JANARDHANAN, Srinivasan, Chennai 600092 (IN)
(74) Representative: Turner, Craig Robert
(86) International application number: PCT/IN2017/050187
(87) International publication number: WO 2017/203538

(56) References cited:
- WO-A1-00/33902
- WO-A1-02/074371
- WO-A1-2006/040585
- DE-U1- 8 908 273
- US-A1- 2004 094 148
- US-A1- 2007 006 876
- US-B1- 6 394 085

## Description

### Field of the Invention:

The present invention relates to an inhaler spacer and to a method of using such an inhaler spacer.

### Background and Prior art:

Inhaled medicaments are commonly prescribed to patients for treating diseases such as asthma and viral induced wheeze. Inhaled medicaments may be administered via a number of means, including metered dose inhalers (MDIs) and nebulizers. MDIs are the most commonly prescribed means for administering inhaled medicaments.

In order to properly use an inhaler, a number of coordinated actions are required (pressing down on the inhaler, breathing in deeply as the medication is released, holding your breath and exhaling). Some patients, for example the very young or elderly, have difficulty completing the required actions in the correct order and at the correct times.

Patients who experience difficulty using an MDI are commonly provided with a spacer device. Generally, spacers are known to reduce coordination difficulties and reduce oropharyngeal deposition thereby considerably increasing the drug delivery in the lungs. Spacer devices connect a drug delivery canister (for example, an MDI) to a mask or mouthpiece. Pressing of the drug canister releases the drug into a chamber of the spacer device. The medicament is held within the chamber by an appropriate means, which upon inhalation, allows the patient to inhale the treatment in his/ her own time through the mouthpiece/ mask.

Use of the spacer device avoids timing issues experienced with MDIs. Indeed, a patient is generally encouraged to breathe "normally" when using a spacer device, rather than to adopt a particular, abnormal, breathing pattern. Further, aerosol is generally issued to the patient from the spacer device more slowly than when issued directly from the MDI, resulting in less of the drug impacting on the back of the mouth and more of the drug reaching the lungs of the patient. For children, spacer devices are particularly useful, allowing them to gain the benefit of inhaled drugs in a way that they can use at home, without the use of hospital nebulizers and masks. In fact, studies have shown that used correctly, these devices can match the efficacy of hospital nebulizers in treatment of asthmatic children.

Various spacer devices are known in the art from a tube spacer with a volume of < 50ml to holding chambers with a volume of up to 750 ml.

WO00/33902 discloses a spacer device for administering orally a medicament by inhalation, the spacer has two conical members made of anti-static material and coupled at their wider ends. A measured dose of medicament is injected into the spacer through an inlet and inhaled from the spacer via the outlet.

WO2006/040585 discloses a spacer having a similar design to that disclosed above, but which further includes a butterfly valve to prevent loss of medicament prior to inhalation.

Other example of commercially known spacer includes AeroChamber Plus^{®} which is shown at www.aerochambervhc.com.

WO02074371 discloses a another spacer with a diffuser portion extending away from an inlet and curving outwardly away from a first axis with increasing distance away from the inlet.

The present inventors have now identified that the spacers described in the prior art and those commercially available to date may not be entirely satisfactory for all uses and users. In particular, spacers tend to be quite bulky and therefore may be inconvenient to carry. Given that patient compliance is important, there exists a need for an improved spacer and have devised one which substantially overcomes some of the problems associated with the known spacer devices.

### Object of the Invention:

An object of the present invention is to provide an inhaler spacer which caters to the patient's need and addresses some of the issues above.

### Summary of the Invention:

According to one aspect of the present invention, there is provided an inhaler spacer as defined in the appended claims. It comprises an inlet for connection to an inhaler and an outlet through which a user can inhale, the inhaler spacer comprising a duct extending between the inlet and the outlet, the duct being defined by a wall which extends around a first axis and comprising a diffuser portion extending along the first axis away from the inlet, the wall of the duct in at least a part of the diffuser portion curving outwardly away from the first axis with increasing distance away from the inlet such that the cross sectional area of the diffuser portion perpendicular to the first axis increases with increased distance from the inlet along the first axis.

According to another aspect of the disclosure not claimed, there is provided an entry section for coupling to an exit section to form an inhaler spacer, wherein the exit section may comprise a reducer portion and the entry section may comprise an inlet and a diffuser portion wherein the wall of at least a part of the diffuser portion may curve outwardly away from the first axis with increasing distance away from the inlet such that the cross sectional area of the diffuser portion perpendicular to the first axis increases with increased distance from the inlet along the first axis.

### Detail Description of the Invention:

The inhaler spacer of the present invention comprises a diffuser portion which, at least in a part, curves outwardly away from the first axis with increasing distance away from the inlet and the inventors of the present invention have surprisingly found that such a shape is advantageous as it increases the Fine Particle Dose (FPD) of medicament which can be drawn from the outlet of the inhaler spacer, for example by inhalation when compared to an inhaler spacer of similar overall length and maximum diameter. An increased FPD may result in increased deposition within the lungs of a patient when the spacer is used to deliver medicament and this can enhance the efficacy of delivery of a medicament, or reduce the required dose. Alternatively, the increased FPD made possible by the new shape of at least a part of the diffuser portion may result in a smaller inhaler spacer being able to deliver FPD performance which is substantially similar to the performance of a larger device. It should be understood that the length and maximum diameter of an inhaler spacer typically determine how easily portable and / manoeuvrable the inhaler spacer is for a user or caregiver. It has been found that reducing one, or both, of these dimensions of a known inhaler spacer results in the FPD of the inhaler spacer being reduced. This means that, although a reduction in size is possible which may improve compliance as the patient is more likely to use the device, the FPD would be reduced and this may adversely affect the therapeutic performance of the inhaler. However, modifying an existing spacer based on the new shape for the diffuser portion of the present invention improves the FPD such that therapeutic performance may be enhanced, or therapeutic performance can be retained while the length and / or maximum diameter are reduced.

The inhaler spacer comprises a duct extending along a first axis between the inlet and the outlet. The duct therefore provides, or defines, a chamber between the inlet and the outlet. The duct substantially surrounds the first axis such that a medicament introduced into the chamber via the inlet is substantially prevented from leaving the inhaler spacer except via the outlet. The duct may have any suitable cross section, but it is preferred that the cross section perpendicular to the first axis is substantially circular as this substantially matches the shape of a plume of medicament ejected from a pMDI.

The inhaler spacer may be fabricated from any suitable material and may include transparent, translucent and / or opaque materials. At least some, possibly all, of the wall of the duct may be fabricated from an antistatic material, for example polyamide. A transparent material for the duct may be preferred to enable a user to see the cloud of medicament within the spacer prior to inhalation and to facilitate visual inspection of the device for cleanliness or foreign objects that may have entered the spacer.

The diffuser portion may be substantially circular cross section perpendicular to the first axis and the radius of the cross section may increase with increased distance from the inlet along the first axis. The increasing cross section causes a plume of medicament emitted from a pMDI through the inlet to slow within the spacer so that it can be more easily inhaled through the outlet. The slowing of the plume may effectively temporarily store the medicament plume within the spacer to help reduce coordination issues which can occur with the use of a pMDI. The shape of the diffuser section is designed to produce a minimal gap between the wall of the duct and the plume of injected medicament, particularly adjacent the inlet. This ensures that there is minimal non-aerosol air in the flow and can also aid evacuation of the chamber during inhalation.

The outward curve of the diffuser portion may be a substantially continuous curve, or may comprise a plurality of curved and / or conical segments that result in an outwardly curving shape of the diffuser portion. For example a sequence of substantially frustoconical sections, each having an increased angular offset from the first axis would produce such a curved shape. Some, or all, of the sections noted above may include a curve. At least two conical segments are required in order to be regarded as a curve. There may be more than three conical segments, or more than four conical segments in the diffuser portion. The conical segments may be separated by tubular segments or constant diameter, but an outwardly curving shape for at least part of the diffuser portion may still be achieved as long as the overall trend is an outward curve in at least a portion of the diffuser portion.

The rate of increase of radius of a circular diffuser portion, or the rate of increase of a dimension of a non-circular diffuser portion, may increase with increased distance from the inlet so that the diffuser flares outwards. The increase in the rate of increase may be substantially continuous, or may be discontinuous along the curve.

The wall of the diffuser portion proximal the inlet may extend at an angle of less than 5 degrees away from the first axis, less than 3 degrees away from the inlet or may extend substantially parallel with the first axis.

The wall of the at least part of the diffuser portion which curves outwardly that is distal from the inlet may extend at an angle of more than 15 degrees away from the first axis, more than 18 degrees away from the first axis or substantially 20 degrees from the first axis.

Then outward curve may comprise the majority, or substantially all, of the diffuser portion in some embodiments. In other embodiments the wall of the duct in at least part of the diffuser portion curves inwardly towards the first axis with increasing distance away from the inlet, the outwardly curving portion being arranged between the inlet and the inwardly curving portion such that, in the diffuser portion having inwardly curving walls, the cross sectional area of the diffuser portion perpendicular to the first axis continues to increase with increased distance from the inlet along the first axis, or at least does not decrease within the diffuser portion.

It should be understood that by the term outwardly curving it is meant that the angular offset of wall (a tangent from the wall at the point being measured) from the first axis increases with distance from the inlet along the first axis. The angular offset may vary between 0 degrees (parallel with the first axis) and 90 degrees (perpendicular to the first axis), although it is preferred that 90 degrees is not reached. It should be further understood that by inwardly curving it is meant that the angular offset of wall from the first axis decreases with distance from the inlet along the first axis. The inward or outward curve is determined by the change in the angular offset of the wall from the first axis moving away from the inlet along the first axis. If the angular offset increases then it is called an outward curve, but if the angular offset decreases then it is an inward curve. If the angular offset does not change over a portion of the first axis the wall would define a cylinder, or a part of a cone.

The maximum angular offset of the wall within the diffuser portion may be less than 60 degrees, less than 45 degrees, may be less than 35 degrees, or less than 30 degrees.

The outward and then inward curve of the duct wall in the diffuser portion may form a substantially continuous curve, such as an elongated 'S' curve. A substantially continuous curve is one which includes substantially no significant discontinuities, such as sharp corners. Discontinuities along the wall of the duct may result in turbulence or settling of the medicament on the wall which reduces the FPD that can be delivered to a patient. Medicament settling of the wall of the inhaler spacer also results in an increased cleaning burden for a user.

The diffuser portion may be substantially rotationally symmetrical about the first axis.

The diffuser portion may extend for between 5 cm and 14 cm along the first axis, or for between 6 cm and 12 cm along the first axis. The outwardly curving part of the diffuser portion may extend for between 2 cm and 12 cm along the first axis. An inwardly curving portion of the diffuser may extend for between 2 cm and 10 cm along the first axis

The minimum cross sectional area of the diffuser portion perpendicular to the first axis may be between about 5 cm² and 9 cm². The maximum cross sectional area of the diffuser portion perpendicular to the first axis may be between about 12 cm² and 50 cm².

The maximum diameter of a substantially circular diffuser portion may be between 4 cm and 8 cm, or between 5 cm and 7 cm.

The inlet of the inhaler spacer may be shaped to receive and retain a mouthpiece of an inhaler, particularly an actuator of a pressurised metered dose inhaler. The inlet may include ribs or other protrusions such that when the actuator mouthpiece is inserted into the inlet air channels exist between the inlet of the spacer and the mouthpiece. These air channels can assist in emptying the spacer during inhalation. It is preferred that the mouthpiece of an inhaler retained in the inlet should be arranged as close to the duct walls as possible. The widest dimension of the mouthpiece of the inhaler may be less than 5 mm smaller, less than 3mm smaller, or substantially equal to the diameter of the duct at the start of the diffuser portion. This helps to reduce the amount of air between the plume of medicament emitted and the duct.

The inhaler spacer may include a reducer portion between the diffuser portion and the outlet. The wall of the duct in the reducer portion may extend towards the first axis with increasing distance away from the inlet such that the cross sectional area of the reducer portion perpendicular to the first axis decreases with increased distance from the inlet along the first axis. The reducer portion may be substantially conical, or the wall of the duct in at least a part of the reducer portion may be continuously curved such that the wall curves inwardly from a wide end of the reducer portion and outwardly near a narrow end of the reducer section.

The inhaler spacer may include a transition portion between the diffuser portion and the reducer portion. The transition portion may comprise a releasable coupling allowing the diffuser portion to be separated from the reducer portion. The releasable coupling may comprise a bayonet type releasable coupling, or could be a friction fit, or press fit coupling, a screw fit coupling, a separate ring clamp or strap coupling or any other type of coupling suitable for joining two parts of a duct together. Including a releasable coupling in the transition portion allows the inhaler spacer to be separated into an entry section, including the diffuser portion and the inlet, and an exit section including the reducer section and an outlet. The separation of the portions facilitates cleaning of the inhaler spacer and also facilitates manufacturing as changes to one section need not impact the other. This means that an entry section including the new diffuser shape can be coupled to an exit section of an existing inhaler spacer.

The outlet may take any suitable form, but may comprise a mouthpiece. The mouthpiece may protrude from the spacer such that a user can place their lips around the mouthpiece to create a substantial seal during inhalation of a medicament from the spacer. The inhaler spacer may further include a movable mouthpiece cover which is coupled to the inhaler spacer and can be moved between a closed position in which mouthpiece cover substantially covers the mouthpiece and an open position in which the mouthpiece is exposed for use.

A valve, for example a one way valve may be provided adjacent the outlet which may close (or be closed) so that a user may be hindered from exhaling into the spacer through the outlet and open (or be opened) to allow inhalation from the inhaler spacer through the outlet. There are many suitable valves known to those skilled in the art, for example a butterfly valve of the type disclosed in WO2006/040585 may be suitable.

The invention also provides a method of preparing a medicament dose for use, the method comprising the steps of:
a) providing a pressurised metered dose inhaler having an inhaler mouthpiece;
b) providing an inhaler spacer, the inhaler spacer being as described above;
c) fitting the inhaler mouthpiece to the inlet of the spacer; and
d) dispensing a dose of medicament into the spacer from the pressurised metered dose inhaler.

Preparing a dose of medicament in this way allows separation of the act of dispensing the dose and the subsequent inhalation of the medicament. If desired, the dose may be held, at least temporarily within the chamber of the spacer with the medicament suspended in the air within the spacer prior to inhalation.

In order to deliver the medicament to a patient the dose may be prepared as above and the patient caused to inhale through the outlet of the inhaler spacer. Substantially simultaneous dispensing of the dose into the spacer and inhalation from the inhaler is also possible.

The invention also extends to an entry section for coupling to an exit section to form an inhaler spacer, the entry section including a diffuser portion and the exit section including a reducer portion. The inhaler spacer may be substantially as described above and the entry section may include an inlet and the wall of at least a part of the diffuser portion may curve outwardly away from the first axis with increasing distance away from the inlet such that the cross sectional area of the diffuser portion perpendicular to the first axis increases with increased distance from the inlet along the first axis.

The invention will now be described by way of example only with reference to the following figures in which:
Figure 1 shows a cross section through a first inhaler spacer;
Figure 2 shows a cross section through a second inhaler spacer;
Figure 3 shows a cross section through the inhaler spacer of Figure 2 coupled to a pMDI actuator; and
Figures 4 and 5 show an inhaler spacer separated into an entry section (Figure 5) and an exit section (Figure 4).

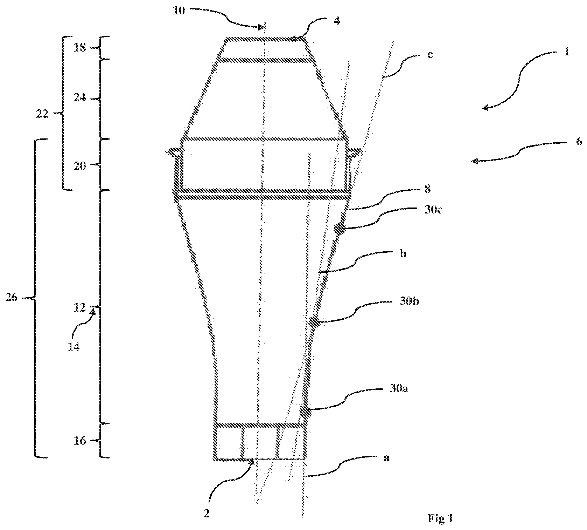

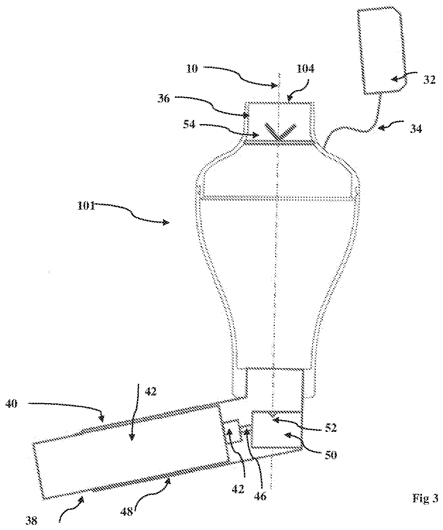

Figure 1 shows an inhaler spacer 1 comprising an inlet 2 for connection to an inhaler, particularly a pMDI (shown in Figure 3) and an outlet 4 through which a user can inhale. The inhaler spacer 1 comprises a duct 6 extending between the inlet 2 and the outlet 4. The duct 6 is defined by a wall 8 which extends around a first axis 10 and comprises a diffuser portion 12 extending along the first axis away from the inlet 2. The wall 8 of the duct 6 in at least a part 14 of the diffuser portion 12 curves outwardly away from the first axis 10 with increasing distance away from the inlet 2 such that the cross sectional area of the diffuser portion 12 perpendicular to the first axis 10 increases with increased distance from the inlet 2 along the first axis 10.

The inlet 2 is located in an inlet portion 16 and the outlet 4 in an outlet portion 18. The diffuser portion 12 forms part of an entry section 26 comprising the inlet portion 16, diffuser portion 12 and a part of a transition portion 20. The transition portion 20 couples the entry section 26 to an exit section 22 which comprises the outlet portion 18 and a reducer portion 24 and a part of the transition portion 20. The inhaler spacer 1 is substantially circular in cross section perpendicular to the first axis 10, although it should be noted that the inlet portion 16, outlet portion 18 and potentially the transition portion 20 may not be exactly circular as they are intended to fit with other elements.

Marked on Figure 1 are some lines a, b and c indicating the direction in which the duct wall 8 extends at particular locations 30a, 30b and 30c in the diffuser portion 12. The lines a, b and c share a letter with their respective locations 30a, 30b and 30c. It can be seen that at an end of the diffusion portion 12 proximal the inlet portion 16 the wall extends substantially parallel with the first axis 10 (30a). As the duct extends along the first axis the angular offset from the first axis 10 of the duct wall 8 within the part 14 of the diffuser portion 12 increases (30b and 30c).

The reducer portion 24 is substantially frustoconical in shape and reduces the diameter of the duct 6 from the transition portion 20 to the outlet portion 18 as the duct 6 extends along the first axis 10.

The Fine Particle Dose (FPD) delivered by an inhaler spacer following the design of the inhaler spacer 1 of Figure 1 was tested and compared to known inhaler spacers. The FPD was defined to be the dose of drug having an aerodynamic particle size below 5µm (allowing inhalation into the lungs) that was drawn from the inhaler spacer as tested by Cascade Impactor. The new inhaler spacer had a diffuser portion 12 that was about 9cm long and varied from having a wall that was substantially parallel with the first axis 10 adjacent the inlet portion 16 to extending away from the first axis 10 at an angle of about 20 degrees at the end distal from the inlet portion 16. This resulted in a diameter increase over the diffuser portion from about 3cm to about 6.3cm.

It can be seen from the table that the new inhaler spacer 1 of Figure 1 achieved a significantly higher FPD than the prior art. This means that the present invention can provide inhaler spacers that have significantly improved FPDs for their size, or the present invention can allow a smaller physical size for a FPD comparable to the prior art inhaler spacers.

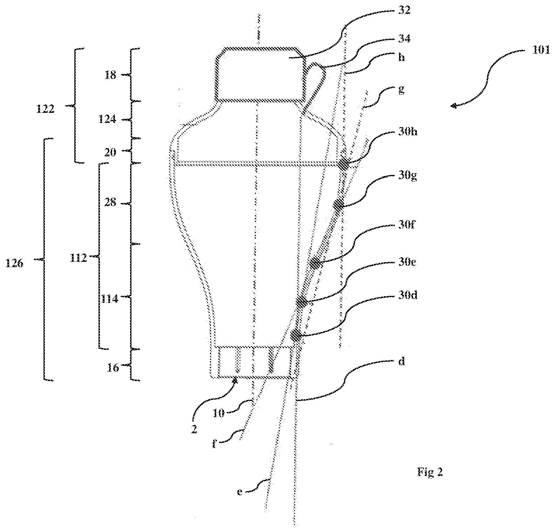

Figure 2 shows an inhaler spacer 101 having a FPD comparable to the prior art inhaler spacers, but having a reduced physical size. Features substantially identical to those of inhaler spacer 1 of Figure 1 are marked with the same reference numeral. Features that are functionally similar to those of the inhaler spacer 1 of Figure 1 are marked with the same reference numeral incremented by 100.

In this case, a key difference between the inhaler spacer 101 and the inhaler spacer 1 is that the part 114 of the diffuser portion 112 that has an outward curve does not occupy substantially the entire diffuser portion 112 of the intake section 126. There is an inwardly curving part 28 of the diffuser portion 112 between the outwardly curving part 114 and the transition portion 20. In the inwardly curving portion the wall 8 of the duct 6 curves inwardly towards the first axis 10 with increasing distance away from the inlet 2. Since this follows the outwardly curving section 14, the inwardly curving section returns the wall to a substantially parallel with the first axis 10. This means that in the inwardly curving section 28 the cross sectional area of the diffuser portion 112 perpendicular to the first axis 10 still increases, or at least does not decrease, with increased distance from the inlet 2 along the first axis 10. The dual curving parts, outward 114 and then inward 28, results in a continuous curve that increases the diameter of the duct, but does not introduce any significant discontinuities, such as sharp corners, into the duct 6.

The reducer portion 124 has a similar continuously curving profile with an initial inward curve moving away from the transition portion 20 and then an outward curve approaching the outlet portion 18 such that the diameter of the duct is reduced without introducing any significant discontinuities, such as sharp corners, into the duct 6.

Marked on Figure 2 are some lines d, e, f, g and h indicating the direction in which the duct wall 8 extends at particular locations 30d, 30e, 30f, 30g and 30h in the diffuser portion 112. The lines d, e, f, g and h share a letter with their respective locations 30d, 30e, 30f, 30g and 30h. It can be seen that at an end of the diffusion portion 112 proximal the inlet portion 16 the wall extends substantially parallel with the first axis 10 (30d). As the duct extends along the first axis the angular offset from the first axis 10 of the duct wall 8 within the part 114 of the diffuser portion 112 increases (30e) to a maximum (30f). The wall 8 then curves inwardly in the part 28 of the diffuser portion 112 such that the angular offset from the first axis is reduced from the maximum (30g) and returns to substantially parallel (30h).

The inhaler spacer 101 also includes a movable mouthpiece cover 32 coupled to the spacer by a tether 34. The mouthpiece cover 32 is adapted to be moved between a closed position in which mouthpiece cover 32 substantially covers the outlet 104 (shown in Figure 3), in this case a mouthpiece 36 (shown in Figure 3) and an open position in which the mouthpiece 36 is exposed for use.

Figure 3 shows the inhaler spacer 101 of Figure 2 coupled to a pMDI inhaler 38 for use by a user. The pMDI inhaler 38 comprises an actuator 40 and a pressurised canister 42 containing a medicament formulation. The canister 42 incudes a valve 44 and valve stem 46 and is operable to dispense under pressure a metered dose medicament formulation through the valve stem 46 when the valve stem 46 is pressed into the valve 44

The actuator 40 comprises a body 48 which houses an actuator block 50 into which the valve stem 46 is fitted. The actuator block 50 includes a conduit to conduct medicament emitted from the valve stem 46 to an outlet 52 and thereby aerosolise the medicament formulation for inhalation.

The outlet portion 18 (shown in Figure2) includes a valve, in this case a butterfly valve 54 which hinders a user from exhaling into the inhaler spacer 101. The outlet portion 18 in this case is in the form of a protruding mouthpiece 36 intended to facilitate a user forming a seal around the outlet 104 with their lips during use.

The results of the comparative tests are presented in Table 1 below:
**Fine Particle Dose (FPD) by Cascade Impactor using different inhaler spacers**
(Using Fluticasone Propionate Metered Dose Inhaler - 110 mcg/dose)

**Table 1**

| **Spacer Used** | **FPD (meg) out of 110 mcg** |
|---|---|
| Zerostat - VT | 73 |
| Aerochamber | 58 |
| Able Disposable Spacer | 35 |
| **Inhaler Spacer of** **Figure 1** | **94** |
| **Inhaler Spacer of** **Figure 2** | **61** |

Table 2 below provides comparative FPD and size data from those tests for various inhaler spacers. In particular, data is included in Table 2 for a reduced size version of the Zerostat VT (the Zerostat VT Small) and data for the inhaler spacers of Figures 1 and 2.

**Table 2**

| **Spacer** | **Volume** | **Length** | **Diameter** | **FPD** |
|---|---|---|---|---|
| **Zerostat VT** | 280 ml | 175mm | 75 mm | 73 mcg |
| **Zerostat VT Small** | 240 ml | 145 mm | 75 mm | 62 mcg |
| **Aerochamber** | 150 ml | 150 mm | 50 mm | 58 mcg |
| **Inhaler Spacer of** **Figure 1** | 240 ml | 175 mm | 65 mm | 94 mcg |
| **Inhaler Spacer of** **Figure 2** | 160 ml | 124 mm | 63 mm | 61 mcg |

It can be seen that the FPD for the Zerostat VT Small is about 11mcg lower than for the full sized version and this is as a result of shortening the inhaler spacer by about 30mm. By comparison, the Inhaler spacer of Figure 2 is a further about 20mm shorter and has a smaller diameter than the Zerostat VT Small, but has a substantially similar FPD.

The tables above demonstrate that, using the present invention, it is possible to create an inhaler spacer having an enhanced FPD for a particular length when compared with prior designs, see for example the comparison between Zerostat VT and the Inhaler Spacer of Figure 1. The tables also shows that it is possible to create a short inhaler spacer, for example one of less than 125mm in length, which has an acceptably high FPD.

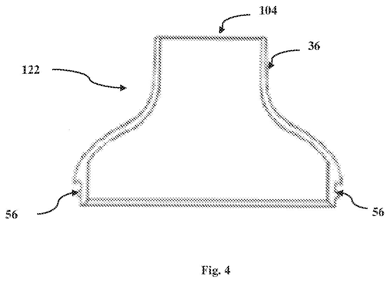

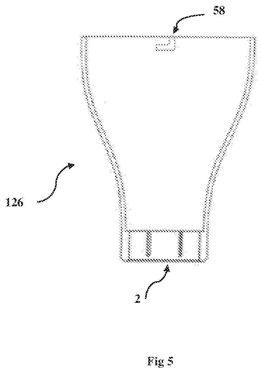

Figures 4 and 5 show the entry section 126 (Figure 5) and exit section 122 (Figure 4) of the inhaler spacer 101 separated from one another using the releasable coupling in the transition section 20. In this case the releasable coupling is provided by a bayonet style coupling comprising one or more protrusions 56 on one part which engage in one or more shaped recesses 58 in the other part. In this inhaler spacer the protrusions 56 are carried by the exit section 122 and the recesses are carried by the entry section 126, although it could be the other way around, or possibly a combination of the two.

## Claims

1. An inhaler spacer (1) comprising an inlet (2) for connection to an inhaler and an outlet (4) through which a user can inhale, the inhaler spacer (1) comprising a duct (6) extending between the inlet (2) and the outlet (4), the duct (6) being defined by a wall (8) which extends around a first axis (10) and comprising a diffuser portion (12) extending along the first axis (10) away from the inlet (2), the wall of the duct (6) in at least a part of the diffuser portion (12) curving outwardly away from the first axis (10) with increasing distance away from the inlet (2) such that the cross sectional area of the diffuser portion (12) perpendicular to the first axis (10) increases with increased distance from the inlet (2) along the first axis (10), wherein the diffuser portion (12) extends between 6 cm and 12 cm along the first axis (10) and has a maximum diameter of between 4 cm and 8 cm, wherein the outlet (4) comprises a protruding mouthpiece, and the spacer includes a movable mouthpiece cover (32) which is coupled to the inhaler spacer (1) and can be moved between a closed position in which mouthpiece cover (32) substantially covers the mouthpiece and an open position in which the mouthpiece is exposed for use, wherein the wall (8) of the diffuser portion (12) proximal the inlet (2) extends at an angle of less than 5 degrees away from the first axis (10) and the wall (8) of the diffuser portion (12) distal from the inlet (2) extends at an angle of more than 15 degrees away from the first axis (10).

2. The inhaler spacer (1) as claimed in claim 1, in which the outward curving of the duct wall (8) in the diffuser portion (12) is a substantially continuous outward curve.

3. The inhaler spacer (1) as claimed in claim 1 or claim 2, in which the diffuser portion (12) has a substantially circular cross section perpendicular to the first axis (10) and the radius of the cross section increases with increased distance from the inlet (2) along the first axis (10) within the outwardly curving portion, optionally in which the rate of increase of radius increases with increased distance from the inlet (2).

4. The inhaler spacer (1) as claimed in any preceding claim, in which the wall (8) of the diffuser portion (12) proximal the inlet extends substantially parallel with the first axis (10).

5. The inhaler spacer (1) as claimed in any preceding claim, in which the wall of the diffuser portion (12) distal from the inlet (2) extends at an angle greater than 18 degrees away from the first axis (10).

6. The inhaler spacer (1) as claimed in any of claims 1 to 4, in which the wall of the duct (6) in at least part of the diffuser portion (12) curves inwardly towards the first axis (10) with increasing distance away from the inlet (2), the outwardly curving portion being arranged between the inlet (2) and the inwardly curving portion (28) such that, in the diffuser portion (12) having inwardly curving walls, the cross sectional area of the diffuser portion (12) perpendicular to the first axis (10) increases with increased distance from the inlet (2) along the first axis (10), optionally in which the outward and then inward curve of the duct wall (8) in the diffuser portion (12) forms a substantially continuous curve.

7. The inhaler spacer (1) as claimed in any preceding claim, comprising one or more of the following features:
(i) in which the diffuser portion (12) is substantially rotationally symmetrical about the first axis (10);
(ii) in which the minimum cross sectional area of the diffuser portion (12) perpendicular to the first axis is between 5 cm2 and 9 cm2;
(iii) in which the maximum cross sectional area of the diffuser portion (12) perpendicular to the first axis is between 20 cm2 and 35 cm2.

8. The inhaler spacer (1) as claimed in any preceding claim, in which the inhaler spacer (1) includes a reducer portion (24) between the diffuser portion (12) and the outlet, the wall of the duct (6) in the reducer portion (24) extending towards the first axis (10) with increasing distance away from the inlet such that the cross sectional area of the reducer portion (24) perpendicular to the first axis (10) decreases with increased distance from the inlet (16) along the first axis (10), optionally in which the wall (8) of the duct (6) in the reducer portion (24) is substantially conical.

9. The inhaler spacer (1) claimed in claim 8, in which the wall (8) of the duct (6) in at least a part of the reducer portion (24) is continuously curved such that the wall (8) curves inwardly from a wide end of the reducer portion (24) and outwardly near a narrow end of the reducer portion (24).

10. The inhaler spacer (1) as claimed in any of claims 8 to 9, in which the inhaler spacer (1) includes a transition portion (20) between the diffuser portion (12) and the reducer portion (24), the transition portion (20) comprising a releasable coupling allowing the diffuser portion (12) to be separated from the reducer portion (24), optionally in which the releasable coupling comprises a bayonet type releasable coupling.

11. The inhaler spacer (1) as claimed in any preceding claim, in which:
(i) a one way valve (54) is provided adjacent the outlet (52) so that a user may be hindered from exhaling into the spacer through the outlet (52), and/or
(ii) in which the inlet (2) is shaped to receive and retain a mouthpiece of an actuator of a pressurised metered dose inhaler.

12. A method of preparing a medicament dose for use, the method comprising the steps of:
a) providing a pressurised metered dose inhaler (38) having an inhaler mouthpiece;
b) providing an inhaler spacer (1) as claimed in any of the preceding claims;
c) fitting the inhaler mouthpiece to the inlet of the spacer (1); and
d) dispensing a dose of medicament into the spacer (1) from the pressurised metered dose inhaler.

13. The inhaler spacer as claimed in claim 1, wherein the maximum diameter of the diffuser portion is between 5 cm and 7 cm.

## Patentansprüche

1. Inhalator-Abstandshalter (1), der einen Einlass (2) zur Verbindung mit einem Inhalator und einen Auslass (4), durch den ein Benutzer einatmen kann, umfasst, wobei der Inhalator-Abstandshalter (1) einen Kanal (6) umfasst, der sich zwischen dem Einlass (2) und dem Auslass (4) erstreckt, wobei der Kanal (6) durch eine Wand (8) definiert ist, die sich um eine erste Achse (10) erstreckt, und einen Diffusorabschnitt (12) umfasst, der sich entlang der ersten Achse (10) von dem Einlass (2) weg erstreckt, wobei sich die Wand des Kanals (6) in mindestens einem Teil des Diffusorabschnitts (12) mit zunehmendem Abstand von dem Einlass (2) von der ersten Achse (10) weg nach außen krümmt, sodass die Querschnittsfläche des Diffusorabschnitts (12) senkrecht zu der ersten Achse (10) mit zunehmendem Abstand von dem Einlass (2) entlang der ersten Achse (10) zunimmt, wobei sich der Diffusorabschnitt (12) zwischen 6 cm und 12 cm entlang der ersten Achse (10) erstreckt und einen maximalen Durchmesser zwischen 4 cm und 8 cm aufweist, wobei der Auslass (4) ein vorstehendes Mundstück umfasst, und der Abstandshalter eine bewegliche Mundstückabdeckung (32) aufweist, die mit dem Inhalator-Abstandshalter (1) gekoppelt ist und zwischen einer geschlossenen Position, in der die Mundstückabdeckung (32) das Mundstück im Wesentlichen abdeckt, und einer offenen Position, in der das Mundstück zur Verwendung freigelegt ist, bewegt werden kann, wobei sich die Wand (8) des Diffusorabschnitts (12) proximal zu dem Einlass (2) in einem Winkel von weniger als 5 Grad von der ersten Achse (10) weg erstreckt und sich die Wand (8) des Diffusorabschnitts (12) distal von dem Einlass (2) in einem Winkel von mehr als 15 Grad von der ersten Achse (10) weg erstreckt.

2. Inhalator-Abstandshalter (1) nach Anspruch 1, wobei die nach außen gerichtete Krümmung der Kanalwand (8) in dem Diffusorabschnitt (12) eine im Wesentlichen kontinuierliche nach außen gerichtete Krümmung ist.

3. Inhalator-Abstandshalter (1) nach Anspruch 1 oder Anspruch 2, wobei der Diffusorabschnitt (12) einen im Wesentlichen kreisförmigen Querschnitt senkrecht zu der ersten Achse (10) aufweist und der Radius des Querschnitts mit zunehmendem Abstand von dem Einlass (2) entlang der ersten Achse (10) innerhalb des nach außen gekrümmten Abschnitts zunimmt, wobei die Zunahmerate des Radius optional mit zunehmendem Abstand von dem Einlass (2) zunimmt.

4. Inhalator-Abstandshalter (1) nach einem der vorhergehenden Ansprüche, wobei sich die Wand (8) des Diffusorabschnitts (12) proximal zu dem Einlass im Wesentlichen parallel zu der ersten Achse (10) erstreckt.

5. Inhalator-Abstandshalter (1) nach einem der vorhergehenden Ansprüche, wobei sich die Wand des Diffusorabschnitts (12) distal von dem Einlass (2) in einem Winkel von mehr als 18 Grad von der ersten Achse (10) weg erstreckt.

6. Inhalator-Abstandshalter (1) nach einem der Ansprüche 1 bis 4, wobei sich die Wand des Kanals (6) in mindestens einem Teil des Diffusorabschnitts (12) mit zunehmendem Abstand von dem Einlass (2) nach innen in Richtung der ersten Achse (10) krümmt, wobei der nach außen gekrümmte Abschnitt zwischen dem Einlass (2) und dem nach innen gekrümmten Abschnitt (28) angeordnet ist, sodass, in dem Diffusorabschnitt (12), der nach innen gekrümmte Wände aufweist, die Querschnittsfläche des Diffusorabschnitts (12) senkrecht zu der ersten Achse (10) mit zunehmendem Abstand von dem Einlass (2) entlang der ersten Achse (10) zunimmt, wobei die Krümmung der Kanalwand (8) nach außen und anschließend nach innen in dem Diffusorabschnitt (12) eine im Wesentlichen kontinuierliche Krümmung bildet.

7. Inhalator-Abstandshalter (1) nach einem der vorhergehenden Ansprüche, der eines oder mehrere der folgenden Merkmale umfasst:
(i) wobei der Diffusorabschnitt (12) im Wesentlichen rotationssymmetrisch um die erste Achse (10) ist;
(ii) wobei die minimale Querschnittsfläche des Diffusorabschnitts (12) senkrecht zu der ersten Achse zwischen 5 cm² und 9 cm² beträgt;
(iii)wobei die maximale Querschnittsfläche des Diffusorabschnitts (12) senkrecht zu der ersten Achse zwischen 20 cm² und 35 cm² beträgt.

8. Inhalator-Abstandshalter (1) nach einem der vorhergehenden Ansprüche, wobei der Inhalator-Abstandshalter (1) einen Reduzierabschnitt (24) zwischen dem Diffusorabschnitt (12) und dem Auslass aufweist, wobei sich die Wand des Kanals (6) in dem Reduzierabschnitt (24) mit zunehmendem Abstand von dem Einlass in Richtung der ersten Achse (10) erstreckt, sodass die Querschnittsfläche des Reduzierabschnitts (24) senkrecht zu der ersten Achse (10) mit zunehmendem Abstand von dem Einlass (16) entlang der ersten Achse (10) abnimmt, wobei die Wand (8) des Kanals (6) in dem Reduzierabschnitt (24) optional im Wesentlichen konisch ist.

9. Inhalator-Abstandshalter (1) nach Anspruch 8, wobei die Wand (8) des Kanals (6) in mindestens einem Teil des Reduzierabschnitts (24) kontinuierlich gekrümmt ist, sodass die Wand (8) von einem breiten Ende des Reduzierabschnitts (24) nach innen und nahe einem schmalen Ende des Reduzierabschnitts (24) nach außen gekrümmt ist.

10. Inhalator-Abstandshalter (1) nach einem der Ansprüche 8 bis 9, wobei der Inhalator-Abstandshalter (1) einen Übergangsabschnitt (20) zwischen dem Diffusorabschnitt (12) und dem Reduzierabschnitt (24) aufweist, wobei der Übergangsabschnitt (20) eine lösbare Kupplung umfasst, die es ermöglicht, dass der Diffusorabschnitt (12) von dem Reduzierabschnitt (24) getrennt wird, wobei die lösbare Kupplung optional eine lösbare Kupplung vom Bajonett-Typ umfasst.

11. Inhalator-Abstandshalter (1) nach einem der vorhergehenden Ansprüche, wobei:
(i) ein Einwegventil (54) neben dem Auslass (52) vorgesehen ist, sodass ein Benutzer daran gehindert werden kann, durch den Auslass (52) in den Abstandshalter auszuatmen, und/oder
(ii) wobei der Einlass (2) so geformt ist, dass er ein Mundstück eines Aktuators eines unter Druck stehenden Dosierinhalators aufnimmt und hält.

12. Verfahren zur Herstellung einer Medikamentendosis zur Verwendung, wobei das Verfahren die folgenden Schritte umfasst:
a) Bereitstellen eines unter Druck stehenden Dosierinhalators (38) mit einem Inhalatormundstück;
b) Bereitstellen eines Inhalator-Abstandshalters (1) nach einem der vorhergehenden Ansprüche;
c) Anbringen des Inhalatormundstücks an dem Einlass des Abstandshalters (1); und
d) Abgeben einer Medikamentendosis in den Abstandshalter (1) aus dem unter Druck stehenden Dosierinhalator.

13. Inhalator-Abstandshalter nach Anspruch 1, wobei der maximale Durchmesser des Diffusorabschnitts zwischen 5 cm und 7 cm beträgt.

## Revendications

1. Espaceur d'inhalateur (1) comprenant une entrée (2) destinée à être raccordée à un inhalateur et une sortie (4) à travers laquelle un utilisateur peut inhaler, l'espaceur d'inhalateur (1) comprenant un conduit (6) s'étendant entre l'entrée (2) et la sortie (4), le conduit (6) étant défini par une paroi (8) qui s'étend autour d'un premier axe (10) et comprenant une partie diffuseur (12) s'étendant le long du premier axe (10) en s'éloignant de l'entrée (2), la paroi du conduit (6) dans au moins une portion de la partie diffuseur (12) s'incurvant vers l'extérieur en s'éloignant du premier axe (10) d'autant plus qu'elle s'éloigne de l'entrée (2) de telle sorte que la superficie de la section transversale de la partie diffuseur (12) perpendiculairement au premier axe (10) augmente d'autant plus qu'elle s'éloigne de l'entrée (2) le long du premier axe (10), ladite partie diffuseur (12) s'étendant entre 6 cm et 12 cm le long du premier axe (10) et présentant un diamètre maximal compris entre 4 cm et 8 cm, ladite sortie (4) comprenant un embout buccal saillant, et l'espaceur comportant un capuchon d'embout buccal (32) mobile qui est couplé à l'espaceur d'inhalateur (1) et peut être déplacé entre une position fermée dans laquelle le capuchon d'embout buccal (32) recouvre sensiblement l'embout buccal et une position ouverte dans laquelle l'embout buccal est exposé en vue de son utilisation, ladite paroi (8) de la partie diffuseur (12) proche de l'entrée (2) s'étendant selon un angle de moins de 5 degrés en s'éloignant du premier axe (10) et ladite paroi (8) de la partie diffuseur (12) distale par rapport à l'entrée (2) s'étendant selon un angle de plus de 15 degrés en s'éloignant du premier axe (10).

2. Espaceur d'inhalateur (1) selon la revendication 1, dans lequel la courbure vers l'extérieur de la paroi de conduit (8) dans la partie diffuseur (12) est une courbure vers l'extérieur sensiblement continue.

3. Espaceur d'inhalateur (1) selon la revendication 1 ou la revendication 2, dans lequel la partie diffuseur (12) a une section transversale perpendiculairement au premier axe (10) qui est sensiblement circulaire et le rayon de la section transversale augmente d'autant plus qu'il s'éloigne de l'entrée (2) le long du premier axe (10) à l'intérieur de la partie incurvée vers l'extérieur ; facultativement dans lequel le taux d'augmentation de rayon augmente d'autant plus qu'il s'éloigne de l'entrée (2).

4. Espaceur d'inhalateur (1) selon l'une quelconque des revendications précédentes, dans lequel la paroi (8) de la partie diffuseur (12) proche de l'entrée s'étend sensiblement parallèlement au premier axe (10).

5. Espaceur d'inhalateur (1) selon l'une quelconque des revendications précédentes, dans lequel la paroi de la partie diffuseur (12) distale par rapport à l'entrée (2) s'étend selon un angle supérieur à 18 degrés en s'éloignant du premier axe (10).

6. Espaceur d'inhalateur (1) selon l'une quelconque des revendications 1 à 4, dans lequel la paroi du conduit (6) dans au moins une portion de la partie diffuseur (12) s'incurve vers l'intérieur vers le premier axe (10) d'autant plus qu'elle s'éloigne de l'entrée (2), la partie incurvée vers l'extérieur étant agencée entre l'entrée (2) et la partie incurvée vers l'intérieur (28) de sorte telle que, dans la partie diffuseur (12) dotée de parois incurvées vers l'intérieur, la superficie de la section transversale de la partie diffuseur (12) perpendiculairement au premier axe (10) augmente d'autant plus qu'elle s'éloigne de l'entrée (2) le long du premier axe (10) ; facultativement dans lequel la courbure vers l'extérieur puis vers l'intérieur de la paroi de conduit (8) dans la partie diffuseur (12) forme une courbure sensiblement continue.

7. Espaceur d'inhalateur (1) selon l'une quelconque des revendications précédentes, comprenant une ou plusieurs des caractéristiques suivantes :
(i) la partie diffuseur (12) est sensiblement symétrique en rotation autour du premier axe (10) ;
(ii) la superficie de la section transversale minimale de la partie diffuseur (12) perpendiculairement au premier axe est comprise entre 5 cm2 et 9 cm2 ;
(iii) la superficie de la section transversale maximale de la partie diffuseur (12) perpendiculairement au premier axe est comprise entre 20 cm2 et 35 cm2.

8. Espaceur d'inhalateur (1) selon l'une quelconque des revendications précédentes, dans lequel l'espaceur d'inhalateur (1) comporte une partie réducteur (24) entre la partie diffuseur (12) et la sortie, la paroi du conduit (6) dans la partie réducteur (24) s'étendant vers le premier axe (10) d'autant plus qu'elle s'éloigne de l'entrée, de telle sorte que la superficie de la section transversale de la partie réducteur (24) perpendiculairement au premier axe (10) diminue d'autant plus qu'elle s'éloigne de l'entrée (16) le long du premier axe (10) ; facultativement dans lequel la paroi (8) du conduit (6) dans la partie réducteur (24) est sensiblement conique.

9. Espaceur d'inhalateur (1) selon la revendication 8, dans lequel la paroi (8) du conduit (6) dans au moins une portion de la partie réducteur (24) est incurvée de manière continue de telle sorte que la paroi (8) s'incurve vers l'intérieur à partir d'une extrémité large de la partie réducteur (24) et vers l'extérieur près d'une extrémité étroite de la partie réducteur (24).

10. Espaceur d'inhalateur (1) selon l'une quelconque des revendications 8 et 9, dans lequel l'espaceur d'inhalateur (1) comporte une partie de transition (20) entre la partie diffuseur (12) et la partie réducteur (24), la partie de transition (20) comprenant un couplage libérable permettant à la partie diffuseur (12) d'être séparée de la partie réducteur (24) ; facultativement dans lequel le couplage libérable comprend un couplage libérable du type à baïonnette.

11. Espaceur d'inhalateur (1) selon l'une quelconque des revendications précédentes, dans lequel :
(i) une valve unidirectionnelle (54) est prévue adjacente à la sortie (52) de sorte qu'il est possible d'empêcher un utilisateur d'exhaler dans l'espaceur à travers la sortie (52), et/ou
(ii) l'entrée (2) est de forme permettant de recevoir et de retenir un embout buccal d'un actionneur d'un inhalateur doseur sous pression.

12. Procédé de préparation d'une dose de médicament en vue de son utilisation, le procédé comprenant les étapes de :
a) fourniture d'un inhalateur doseur sous pression (38) ayant un embout buccal d'inhalateur ;
b) fourniture d'un espaceur d'inhalateur (1) selon l'une quelconque des revendications précédentes ;
c) montage de l'embout buccal d'inhalateur sur l'entrée de l'espaceur (1) ; et
d) distribution d'une dose de médicament dans l'espaceur (1) à partir de l'inhalateur doseur sous pression.

13. Espaceur d'inhalateur selon la revendication 1, dans lequel le diamètre maximal de la partie diffuseur est compris entre 5 cm et 7 cm.
